# EUROPÄISCHE PATENTSCHRIFT

(11) **EP 3 071 964 B1**
(45) Veröffentlichungstag und Bekanntmachung des Hinweises auf die Patenterteilung: **08.04.2020**
(21) Anmeldenummer: 14799442.0
(22) Anmeldetag: 17.11.2014
(51) Int. Cl.: G01N 33/28

(54) **VORRICHTUNG UND VERFAHREN ZUR DETEKTION VON GAS**
APPARATUS AND METHOD FOR DETECTING GAS
DISPOSITIF ET PROCÉDÉ DE DÉTECTION DE GAZ

(30) Priorität: 20.11.2013 DE 102013112823
(43) Veröffentlichungstag der Anmeldung: 28.09.2016
(73) Patentinhaber: Maschinenfabrik Reinhausen GmbH, 93059 Regensburg (DE)
(72) Erfinder: HOLLUNDER, Sebastian, 61389 Schmitten - Niederreifenberg (DE); KUBICZEK, Martin, 65719 Hofheim (DE); SCHÜBEL, Jürgen, 61440 Oberursel (DE); VIERECK, Karsten, 93049 Regensburg (DE)
(86) Internationale Anmeldenummer: PCT/EP2014/074745
(87) Internationale Veröffentlichungsnummer: WO 2015/074990

(56) Entgegenhaltungen:
- EP-A1- 0 179 296
- EP-A1- 2 233 922
- DD-A1- 254 827
- DE-B3-102010 008 066
- JP-A- S57 156 536
- US-B1- 6 526 805

## Beschreibung

Die Erfindung betrifft eine Vorrichtung und ein Verfahren zur Detektion von Gas in mit Isoliermedium gefüllten Hochspannungsgeräten, insbesondere Hochspannungstransformatoren.

WO 2011/120113 A1 beschreibt ein System und ein Verfahren zur Überwachung von Gasen in mit Öl gekühlten Leistungstransformatoren. Das System besteht dabei aus einem Stab und einem Hauptgehäuse. Der Stab weist zwei im Inneren verlaufende Leitungen auf und ist im Öl des Leistungstransformators positioniert. Die Leitungen sind über zwei Ölkammern und eine Pumpe miteinander verbunden, sodass das Öl aus dem Leistungstransformator über eine Leitung zur ersten Ölkammer angesaugt und im Anschluss über die zweite Ölkammer durch die andere Leitung wieder in den Leistungstransformator eingeleitet werden kann. Die Pumpe ist dabei in einem Leitungsabschnitt zwischen den Ölkammern angeordnet. Vor der Pumpe befindet sich ein zusätzlicher Bereich mit einem Temperaturfühler und einem Feuchtigkeitsensor. Beide Ölkammern weisen eine Wandung auf, die aus einem semipermeablen Werkstoff besteht. Durch diese Wandung können Gase, die im Öl des Leistungstransformators enthalten sind, in das Innere des Hauptgehäuses abwandern. Ein zusätzlicher Gassensor ermittelt die Gase, die sich im Hauptgehäuse des Systems ansammeln. Zusätzlich sind am Gehäuse zwei Ventile mit jeweils einem Filter angeordnet. Eines der Ventile wird zum Ansaugen von Luft aus der Umgebung mittels einer Pumpe genutzt. Durch das zweite Ventil wird die Luft aus dem Inneren des Hauptgehäuses abgelassen. Das System wird über eine Steuerung gesteuert.

Dieses bekannte System ist sehr aufwendig aufgebaut. Die Vielzahl der verwendeten Einzelteile gestaltet das System nicht nur teuer, sondern auch wartungsintensiv. Die Ventile für den Luftaustausch verschleißen besonders schnell und bilden damit eine Schwachstelle im System. Die flächig ausgebildete Membran kann bei plötzlichem Druckanstieg und insbesondere beim Evaluieren des Trägergases besonders schnell brechen.

US 6 526 805 B1 beschreibt eine Vorrichtung zur kontinuierlichen Analyse und Messung des Gehalts an Gaskomponenten in einer Isolierflüssigkeit im wesentlichen auf Echtzeitbasis. Diese Vorrichtung umfasst eine Gasextraktionseinheit zur Trennung der Gase von der Flüssigkeit, einen Infrarot-Gasanalysator zur Bestimmung der Konzentration der einzelnen Gaskomponenten, eine Gaspumpe zum Umwälzen der Gase zur Gasextraktionseinheit in einer geschlossenen Schleife und eine Kalibrierungseinrichtung für den Infrarot-Gasanalysator. Die Gasextraktionseinheit umfasst eine gasdurchlässige Polymermembran und eine Gaskammer mit einem Gasstromeinlass und einen Gasstromauslass. Der Infrarot-Gasanalysator umfasst eine Infrarotquelle, eine Gaszelle mit einem Gaseinlass und Gasauslass und einen Quad-Detektor mit extrem schmalbandigen optischen Infrarotfiltern. Die Kalibrierungseinrichtung umfasst eine Ventileinrichtung zum Ausspülen der Gaskomponenten mit Luft und zum Rücksetzen der Infrarotquelle auf Null. Die Ventileinrichtung umfasst ein erstes Dreiwegeventil, das zwischen der Gaspumpe und der Gasextraktionseinheit sitzt, und ein zweites Dreiwegeventil, das zwischen der Gaszelle und der Gaspumpe sitzt.

US 8 075 675 B2 beschreibt eine Vorrichtung zum Extrahieren von Gas aus einer Flüssigkeit. Diese Vorrichtung umfasst ein Gehäuse, eine Trennmembran in dem Gehäuse, einen porösen Membranträger, eine Ölpumpe, eine Gaspumpe, einen Gasverteiler und ein Analyseinstrument. Das Gehäuse definiert einen Fluidpfad und einen Gaspfad, der vom Fluidpfad isoliert ist, und weist einen Einlass in den Fluidpfad, einen Auslass aus dem Fluidpfad, einen Einlass in den Gaspfad und einen Auslass aus dem Gaspfad auf. Die Trennmembran trennt den Fluidpfad von dem Gaspfad und umfasst ein Fluorsilikonelement, das undurchlässig für die Flüssigkeit und durchlässig für das Gas ist. Die Trennmembran hat eine erste Seite, die zum Fluidpfad weist, und eine zweite Seite, die zum Gaspfad weist. Die Trennmembran bildet eine abgeflachte Scheibe, die am Rand dicker und in der Mitte dünner ist. Der Membranträger weist zur ersten Seite der Trennmembran. Der Fluidpfad enthält die erste Seite der Trennmembran. Die Ölpumpe fördert die Flüssigkeit durch den Fluidpfad. Die Gaspumpe fördert ein Trägergas und das aus der Flüssigkeit gelöste Gas durch den Gaspfad. Das Analyseinstrument ist über den Gasverteiler an den Gaspfad angeschlossen. Der Gasverteiler wird in US 6 391 096 B1 ausführlich beschrieben und umfasst sieben Steuerventile.

Ferner ist aus der JP S57 156536 A eine Vorrichtung zum Messen von Wasserstoff in Öl mit einer schlauchförmigen Membran bekannt.

Aufgabe der Erfindung ist es, eine Vorrichtung zur Detektion von Gasmolekülen in einem mit Isoliermedium gefüllten Hochspannungsgerät bereitzustellen, die kostengünstig, wartungsfreundlich sowie verschleißfrei aufgebaut ist, sowie ein Verfahren zur Detektion von Gasen in mit Flüssigkeiten gefüllten Hochspannungsgeräten anzugeben, welches einen sicheren Betrieb der Vorrichtung gewährleistet.

Diese Aufgabe wird durch die Gegenstände der unabhängigen Ansprüche gelöst. Vorteilhafte Weiterbildungen sind in den Unteransprüchen beschrieben.

Die Erfindung schlägt gemäß einem ersten Aspekt eine Vorrichtung zur Detektion von Gas in einem mit einem Isoliermedium gefüllten Hochspannungsgerät laut Anspruch 1 vor.

Durch die Verwendung von zwei Pumpen und das Weglassen von Ventilen ist die vorgeschlagene Vorrichtung besonders verschleißfrei und damit wartungsfreundlich. Ein weiterer Vorteil liegt darin, dass nicht das Isoliermedium, sondern das Trägergas umgewälzt wird. Dadurch ist es möglich, vor dem Anreichern und Detektieren frisches Trägergas in die Vorrichtung einzuleiten und nach der Detektion aus der Vorrichtung abzuführen.

Das Hochspannungsgerät kann nach Bedarf auf beliebige Art und Weise ausgebildet sein, beispielsweise als Hochspannungstransformator, Leistungstransformator, Laststufenschalter, Leistungsschalter, Kondensatordurchführung oder ein anderes ölgefülltes elektrisches Betriebsmittel.

Das Isoliermedium kann nach Bedarf auf beliebige Art und Weise ausgebildet sein, beispielsweise als Isolieröl bzw. Esterflüssigkeit.

Das zu detektierende Gas kann nach Bedarf auf beliebige Art und Weise ausgebildet sein und beispielsweise wenigstens eine Kohlenwasserstoffverbindung und/oder andere Gasmoleküle und/oder andere Gasatome enthalten.

Die semipermeable Membran kann nach Bedarf auf beliebige Art und Weise ausgebildet sein und beispielsweise zumindest teilweise aus Teflon bestehen.

Die Pumpen können nach Bedarf auf beliebige Art und Weise ausgebildet sein, beispielsweise als Membranpumpen.

Es kann vorgesehen sein, dass der Einlass eine erste Leitung aufweist.

Vorzugsweise sitzt in der Leitung ein Filter.

Es kann vorgesehen sein, dass die Membran zumindest teilweise rohrartig und/oder zumindest teilweise schlauchartig ausgebildet ist.

Es kann vorgesehen sein, dass die Membran zumindest teilweise spiralförmig und/oder zumindest teilweise mäanderförmig und/oder zumindest teilweise wendelförmig ausgebildet ist.

Es kann vorgesehen sein, dass der Auslass eine zweite Leitung aufweist.

Vorzugsweise ist die zweite Pumpe in der zweiten Leitung angeordnet, sie kann aber auch in der ersten Leitung angeordnet sein.

Es kann vorgesehen sein, dass eine Messkammer vorgesehen ist, in der der Gassensor angeordnet ist.

Es kann vorgesehen sein, dass in der Messkammer wenigstens ein Thermoelement und/oder wenigstens ein Temperatursensor angeordnet ist.

Es kann vorgesehen sein, dass
- der Temperatursensor und das Thermoelement mit einer Steuereinrichtung verbunden sind;
- das Thermoelement anhand der Messungen des Temperatursensors gesteuert wird. Es kann vorgesehen sein, dass die Messkammer mit der ersten Leitung und über die Membran und die zweite Leitung mit dem Auslass verbunden ist.

Es kann vorgesehen sein, dass die Messkammer zwischen der ersten Leitung und der Membran angeordnet ist.

Es kann vorgesehen sein, dass das Thermoelement als Peltier-Element ausgebildet ist. Es kann vorgesehen sein, dass die Messkammer im Inneren mit einem inerten Material, Platin oder Gold ausgekleidet ist.

Es kann vorgesehen sein, dass die zweite Pumpe in der ersten Leitung oder in der zweiten Leitung sitzt.

Es kann vorgesehen sein, dass
- falls die zweite Pumpe in der ersten Leitung sitzt, dann hat sie ein Aspektverhältnis, das größer als das der zweiten Leitung ist und/oder das größer als 100 oder 200 oder 500 oder 1000 oder 2000 oder 5000 oder 10.000 ist, und/oder hat sie einen Strömungswiderstand, der größer als der der zweiten Leitung ist und/oder der dem einer Leitung mit konstantem Innendurchmesser und einem Aspektverhältnis entspricht, das größer als 100 oder 200 oder 500 oder 1000 oder 2000 oder 5000 oder 10.000 ist;
- falls die zweite Pumpe in der zweiten Leitung sitzt, dann hat sie ein Aspektverhältnis, das größer als das der ersten Leitung ist und/oder das größer als 100 oder 200 oder 500 oder 1000 oder 2000 oder 5000 oder 10.000 ist, und/oder hat sie einen Strömungswiderstand, der größer als der der ersten Leitung ist und/oder der dem einer Leitung mit konstantem Innendurchmesser und einem Aspektverhältnis entspricht, das größer als 100 oder 200 oder 500 oder 1000 oder 2000 oder 5000 oder 10.000 ist.

Das Aspektverhältnis einer Leitung ist dabei das Verhältnis aus ihrer Länge und ihrem Innendurchmesser.

Jede Leitung kann nach Bedarf auf beliebige Art und Weise ausgebildet sein und beispielsweise über ihre Länge einen konstanten Innendurchmesser haben. Alternativ kann in einer Leitung mit einem kleineren Aspektverhältnis wenigstens eine Drossel oder Düse sitzen, die bevorzugt derart dimensioniert ist, dass der Strömungswiderstand dieser Leitung dem einer Leitung mit einem der angegebenen größeren Aspektverhältnisse entspricht.

Es kann vorgesehen sein, dass die erste Pumpe in einer ersten Verbindungsleitung, die einen Ausgang der Messkammer mit einem Eingang der Membran verbindet, oder in einer zweiten Verbindungsleitung sitzt, die einen Ausgang der Membran mit einem Eingang der Messkammer verbindet.

Es kann vorgesehen sein, dass die erste Leitung zwischen der ersten Pumpe und der Membran in eine erste Verbindungsleitung, die einen Ausgang der Messkammer mit einem Eingang der Membran verbindet, oder zwischen der ersten Pumpe und der Messkammer in eine zweite Verbindungsleitung mündet, die einen Ausgang der Membran mit einem Eingang der Messkammer verbindet.

Die Erfindung schlägt gemäß einem zweiten Aspekt ein Verfahren zur Detektion von Gas in einem mit einem Isoliermedium gefüllten Hochspannungsgerät laut Anspruch 12 vor.

Es kann vorgesehen sein, dass beim Spülen das Trägergas durch eine erste Leitung angesaugt, durch die Messkammer und die Membran befördert und durch eine zweite Leitung ausgeblasen wird.

Es kann vorgesehen sein, dass das Trägergas über einen Filter angesaugt wird.

Es kann vorgesehen sein, dass die Menge und/oder Art des Gases in der Messkammer bestimmt werden, bevor und/oder nachdem das Trägergas heraus befördert wurde.

Es kann vorgesehen sein, dass die Menge und/oder Art des Gases in der Messkammer bestimmt werden, bevor und/oder nachdem das Trägergas befördert wurde.

Mit jeder der vorgeschlagenen Vorrichtungen kann beispielsweise eines der vorgeschlagenen Verfahren durchgeführt werden.

Vorzugsweise kann jede der vorgeschlagenen Vorrichtungen derart ausgebildet sein und/oder dazu dienen und/oder dafür geeignet sein, dass sie eines der vorgeschlagenen Verfahren ausführt und/oder ausführen kann.

Die Ausführungen und Erläuterungen zu einem der Aspekte der Erfindung, insbesondere zu einzelnen Merkmalen dieses Aspektes, gelten entsprechend auch analog für die anderen Aspekte der Erfindung.

Im Folgenden werden Ausführungsformen der Erfindung beispielhaft anhand der beigefügten Zeichnungen näher erläutert. Die daraus hervorgehenden einzelnen Merkmale sind jedoch nicht auf die einzelnen Ausführungsformen beschränkt, sondern können mit weiter oben beschriebenen einzelnen Merkmalen und/oder mit einzelnen Merkmalen anderer Ausführungsformen verbunden und/oder kombiniert werden. Die Einzelheiten in den Zeichnungen sind nur erläuternd, nicht aber beschränkend auszulegen. Die in den Ansprüchen enthaltenen Bezugszeichen sollen den Schutzbereich der Erfindung in keiner Weise beschränken, sondern verweisen lediglich auf die in den Zeichnungen gezeigten Ausführungsformen.

Die Zeichnungen zeigen in
- FIG. 1: eine erste Ausführungsform einer Vorrichtung zur Detektion von Gas;
- FIG. 2: eine zweite Ausführungsform der Vorrichtung;
- FIG. 3: eine dritte Ausführungsform der Vorrichtung;
- FIG. 4: eine vierte Ausführungsform der Vorrichtung;
- FIG. 5: ein Verfahren zur Detektion von Gas.

Für gleiche oder gleich wirkende Elemente der Erfindung werden identische Bezugszeichen verwendet. Ferner werden der Übersicht halber nur Bezugszeichen in den einzelnen Zeichnungen dargestellt, die für die Beschreibung der jeweiligen Zeichnung erforderlich sind. Die dargestellten Ausführungsformen stellen lediglich Beispiele dar, wie die erfindungsgemäße Vorrichtung bzw. wie das erfindungsgemäße Verfahren ist bzw. sein kann und stellen somit keine abschließende Begrenzung der Erfindung dar.

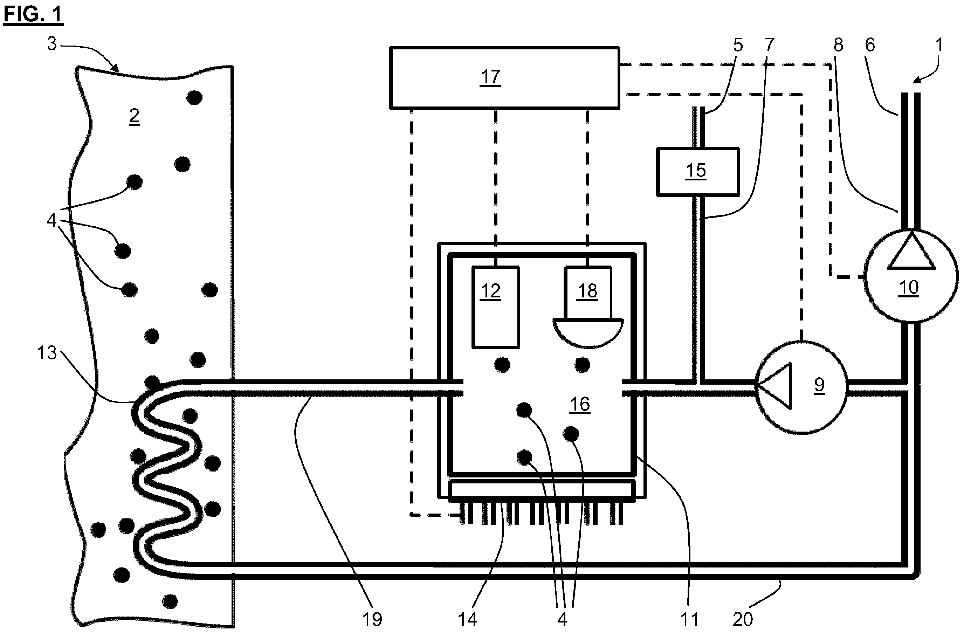

In FIG. 1 ist eine erste Ausführungsform einer Vorrichtung 1 zur Detektion von Gasmolekülen, Ionen oder Gasen 4 in einem Hochspannungsgerät 3 schematisch dargestellt, welches mit einer Flüssigkeit oder einem Isoliermedium 2 gefüllt ist. Das Hochspannungsgerät 3 kann als Hochspannungstransformator, Leistungstransformator, Laststufenschalter, Leistungsschalter oder Kondensatordurchführung ausgebildet sein. Die Vorrichtung 1 weist eine Membran oder Kapillare 13 auf, die aus mindestens einem semipermeablen Werkstoff besteht und rohrartig oder schlauchartig aufgebaut ist. Die rohrartige Membran 13 kann beliebig ausgeformt sein, beispielsweise spiralförmig und/oder wendelförmig und/oder mäanderförmig. Durch diese vorteilhafte Ausgestaltung der Membran 13, ist diese für besonders hohe Drücke geeignet. Die Membran 13 befindet sich im Hochspannungsgerät 3 oder zumindest in einem Teil des Hochspannungsgerätes, dem das Isoliermedium 2 zugänglich ist. Somit kann die Membran 13 in einem Buchholzrelais, einer Leitung der Kühlung, etc. angeordnet sein. Durch die rohrartige Ausgestaltung und den in eine Richtung gasdurchlässigen (semipermeablen) Werkstoff können Moleküle des Gases 4 in einen Kreislauf der Vorrichtung 1 gelangen.

Die Membran 13 ist an einem Ende, das ihren Eingang bildet, über eine erste Verbindungsleitung 19 mit dem Ausgang einer Messkammer 11 und an einem anderen Ende, das ihren Ausgang bildet, über eine zweite Verbindungsleitung 20 mit dem Eingang der Messkammer 11 verbunden. Die Messkammer 11 weist ein Thermoelement 14, das beispielsweise ein Peltier-Element ist, auf, mit dessen Hilfe eine Temperierung der Messkammer 11 durchgeführt wird. Zusätzlich sind in der Messkammer 11 ein Gassensor 12 und ein Temperatursensor 18 angeordnet.

Die Messkammer 11 ist im Inneren mit einem inerten Material, wie z.B. Gold, ausgekleidet bzw. beschichtet. Diese Beschichtung bietet den Vorteil, dass sich die Gase 4 nicht im Inneren niederschlagen bzw. kondensieren und nichtreproduzierbar anlagern, dabei zumindest eine polare, physikalische Bindung eingehen und damit in der Gesamtgasbilanz fehlen können, womit falsche Werte im Vergleich zu einer Laboranalyse gemessen würden.

Weiterhin ist die Messkammer 11 über eine erste Leitung 7, die in die zweite Verbindungsleitung 20 mündet, mit einem Einlass 5 verbunden. Vor dem Einlass 5 ist ein Filter 15 angeordnet. Die erste Leitung 7 weist ein besonders hohes Aspektverhältnis zwischen der eigenen Länge und dem Querschnitt auf. Hierdurch wird erreicht, dass der Druck im Inneren der Vorrichtung 1 dem Druck in der Umgebung, aus der ein Trägergas 16 verwendet wird, entspricht. Da der Druck im Inneren des Hochspannungsgerätes 3 stets höher ist als der Druck der Umgebung und somit auch der Druck in der Vorrichtung, gelangen die im Isoliermedium 2 gelösten Gase 4 in den Kreislauf der Vorrichtung 1 durch die semipermeable Membran 13.

Die Messkammer 11 ist außerdem mit einer ersten Pumpe 9 verbunden, die in der zweiten Verbindungsleitung 20 zwischen der Membran 13 und der ersten Leitung 7 sitzt. Diese befördert das Trägergas 16 durch die Verbindungsleitungen 19, 20, die Messkammer 11 und die Membran 13, sodass ein Kreislauf entsteht und das Trägergas 16 mit den Gasen 4 aus dem Isoliermedium 2 angereichert wird. In ausgeschaltetem Zustand hat die erste Pumpe 9 die Funktion eines geschlossenen Ventils.

Die Vorrichtung 1 weist zusätzlich eine zweite Pumpe 10 auf, die einerseits über eine zweite Leitung 8, die zwischen der Membran 13 und der ersten Pumpe 9 in die zweite Verbindungsleitung 20 mündet, mit einem Auslass 6 und andererseits über die erste Leitung 7, die Messkammer 11 und die Membran 13 mit dem Einlass 5 verbunden ist. Auch die zweite Pumpe 10 übernimmt im ausgeschalteten Zustand die Funktion eines geschlossenen Ventils. Während des Betriebes der ersten Pumpe 9 ist die zweite Pumpe 10 stets ausgeschaltet. Während des Betriebes der zweiten Pumpe 10 ist die erste Pumpe 9 ausgeschaltet.

Wird nun die erste Pumpe 9 eingeschaltet, kommt es zu einer wiederholten Beförderung bzw. Umwälzung des Trägergases 16 durch die Messkammer 11 und die Membran 13. Dabei wird Trägergas 16 mit den Gasen 4, welche durch die semipermeable Membran 13 wandern und somit aus dem Isoliermedium 2 lösen, angereichert, bis die durch das Trägergas 16 aufzunehmende Menge an Gasen 4 nicht mehr signifikant zunimmt.

Wird die zweite Pumpe 10 eingeschaltet, kommt es zu einem Austausch des Trägergases 16 in der Vorrichtung 1. Dieses wird über den Einlass 5 eingesaugt und durch die Messkammer 11 und die Membran 13 zum Auslass 6 weiter geleitet. Der Gassensor 12, der Temperatursensor 18 und das Thermoelement 14 in der Messkammer 11 sowie die erste und die zweite Pumpe 9, 10 sind mit einer zentralen Steuereinrichtung 17 verbunden. Die Steuerung des Thermoelementes 14 erfolgt anhand der Messungen des Temperatursensors 18.

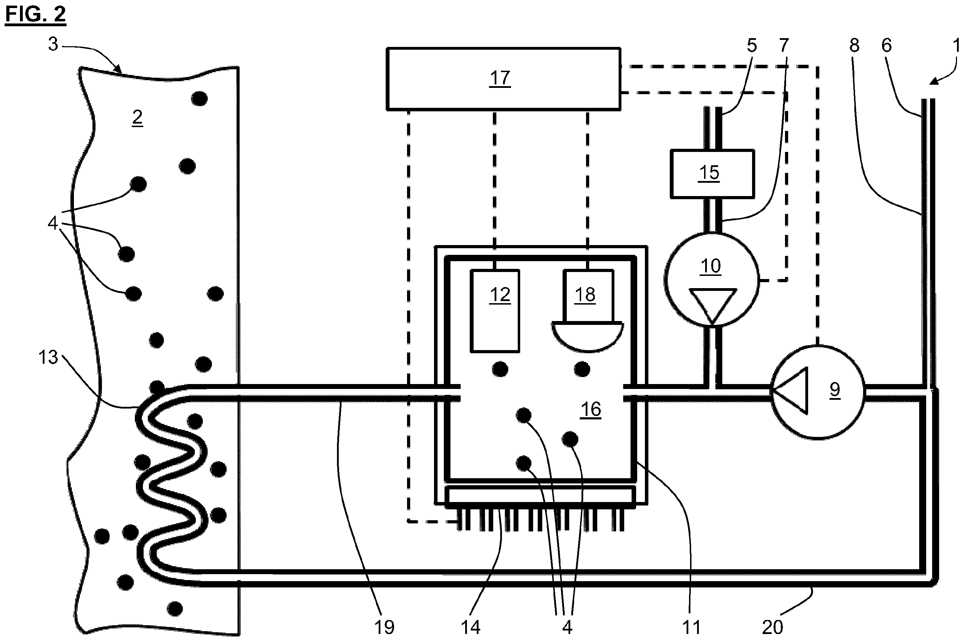

In FIG. 2 ist eine zweite Ausführungsform der Vorrichtung 1 schematisch dargestellt. Diese Ausführungsform ähnelt der ersten Ausführungsform, sodass im Folgenden vor Allem die Unterschiede näher erläutert werden.

Bei dieser Ausführungsform sitzt die zweite Pumpe 10 nicht wie bei der ersten Ausführungsform in der zweiten Leitung 8, sondern in der ersten Leitung 7 und ist folglich einerseits mit dem Einlass 5 und andererseits über die Messkammer 11, die Membran 13 und die zweite Leitung 8 mit dem Auslass 6 verbunden. Außerdem weist nicht wie bei der ersten Ausführungsform die erste Leitung 7, sondern die zweite Leitung 8 ein besonders hohes Aspektverhältnis zwischen der eigenen Länge und dem Querschnitt auf.

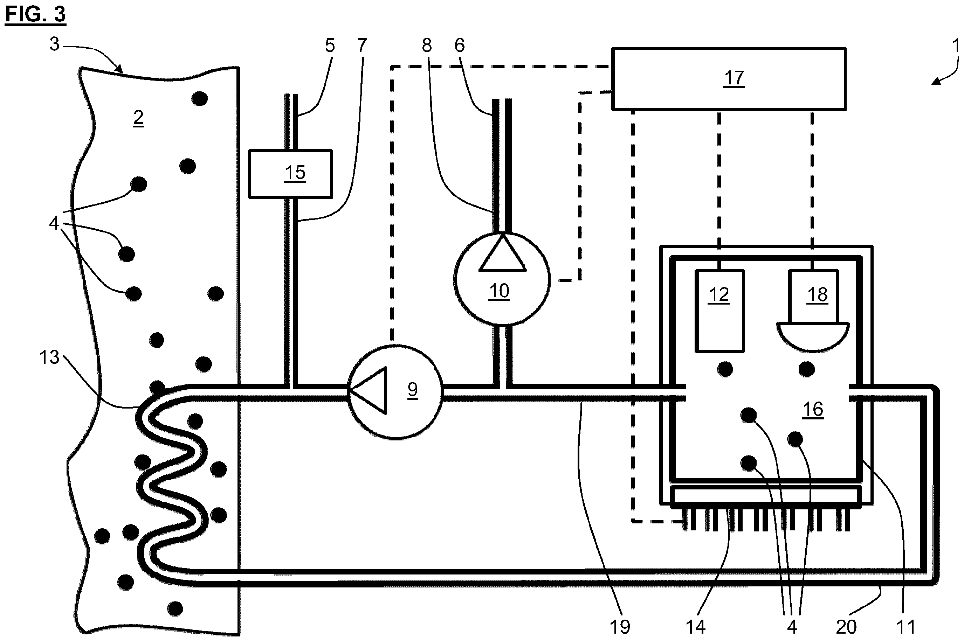

In FIG. 3 ist eine dritte Ausführungsform der Vorrichtung 1 schematisch dargestellt. Diese Ausführungsform ähnelt der ersten Ausführungsform, sodass im Folgenden vor Allem die Unterschiede näher erläutert werden.

Bei dieser Ausführungsform sitzt die erste Pumpe 9 nicht wie bei der ersten Ausführungsform in der zweiten Verbindungsleitung 20, sondern in der ersten Verbindungsleitung 19. Außerdem mündet die ersten Leitung 7 zwischen der ersten Pumpe 9 und der Membran 13 und die zweite Leitung 8 zwischen der Messkammer 11 und der ersten Pumpe 9 in die erste Verbindungsleitung 19.

Zum Spülen saugt folglich die zweite Pumpe 10 das Trägergas über den Einlass 5 ein und befördert es durch den Filter 15, die erste Leitung 7, den stromabwärts gelegenen Teil der ersten Verbindungsleitung 19, die Membran 13, die zweite Verbindungsleitung 20, die Messkammer 11 und den stromaufwärts gelegenen Teil der ersten Verbindungsleitung 19 zum Auslass 6. Zum Anreichern wälzt folglich die erste Pumpe 9 das Trägergas durch den stromabwärts gelegenen Teil der ersten Verbindungsleitung 19, die Membran 13, die zweite Verbindungsleitung 20, die Messkammer 11 und den stromaufwärts gelegenen Teil der ersten Verbindungsleitung 19 um.

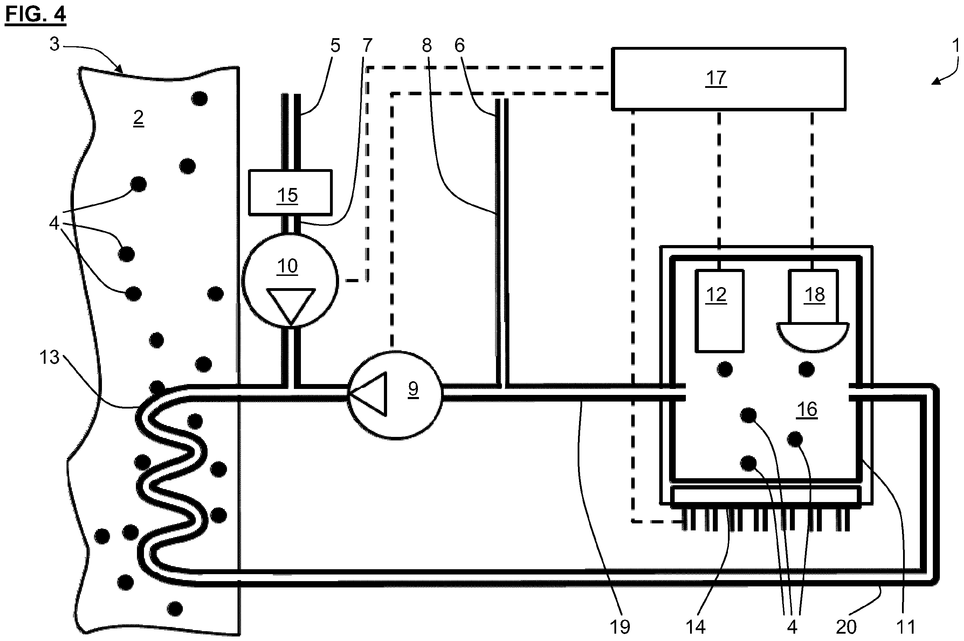

In FIG. 4 ist eine vierte Ausführungsform der Vorrichtung 1 schematisch dargestellt. Diese Ausführungsform ähnelt der dritten Ausführungsform, sodass im Folgenden vor Allem die Unterschiede näher erläutert werden.

Bei dieser Ausführungsform sitzt die zweite Pumpe 10 nicht wie bei der dritten Ausführungsform in der zweiten Leitung 8, sondern in der ersten Leitung 7 und ist folglich einerseits mit dem Einlass 5 und andererseits über die Membran 13, die Messkammer 11 und die zweite Leitung 8 mit dem Auslass 6 verbunden. Außerdem weist nicht wie bei der dritten Ausführungsform die erste Leitung 7, sondern die zweite Leitung 8 ein besonders hohes Aspektverhältnis zwischen der eigenen Länge und dem Querschnitt auf.

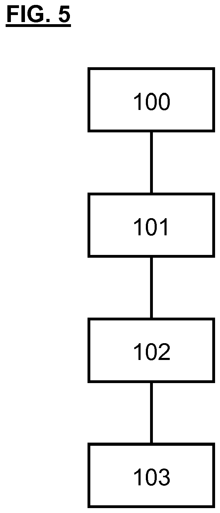

In FIG. 5 ist ein Ablaufdiagramm für eine bevorzugte Ausführungsform eines Verfahrens zur Detektion von Gasen 4 in einem mit einer Flüssigkeit 2 gefüllten Hochspannungsgerät 3 dargestellt, wobei das Verfahren mittels der Vorrichtung 1, die gemäß der ersten, zweiten, dritten oder vierten Ausführungsform ausgebildet ist, ausgeführt wird.

Schritt 100: Zunächst wird die Vorrichtung 1 gespült. Während des Betriebes der zweiten Pumpe 10 ist die erste Pumpe 9 ausgeschaltet und bildet somit ein geschlossenes Ventil. Über den Einlass 5, also den Filter 15 und die erste Leitung 7, wird das Trägergas 16 angesaugt.

Bei der ersten Ausführungsform durchquert dann das Trägergas 16 die Messkammer 11 und die Membran 13, bis es über die zweite Pumpe 10 und die zweite Leitung 8 zum Auslass 6 gelangt.

Bei der zweiten Ausführungsform gelangt dann das Trägergas 16 vom Einlass 5 über den Filter 15, die zweite Pumpe 10 und die erste Leitung 7 zum stromaufwärts gelegenen Teil der zweiten Verbindungsleitung 20 und durchquert die Messkammer 11 und die Membran 13, bis es über die zweite Leitung 8 zum Auslass 6 gelangt.

Bei der dritten Ausführungsform durchquert dann das Trägergas 16 die Membran 13 und die Messkammer 11, bis es über die zweite Pumpe 10 und die zweite Leitung 8 zum Auslass 6 gelangt.

Bei der vierten Ausführungsform gelangt dann das Trägergas 16 vom Einlass 5 über den Filter 15, die zweite Pumpe 10 und die erste Leitung 7 zum stromaufwärts gelegenen Teil der ersten Verbindungsleitung 19 und durchquert die Membran 13 und die Messkammer 11, bis es über die zweite Leitung 8 zum Auslass 6 gelangt.

Schritt 101: Nach einer vorgegebenen Zeit oder in Abhängigkeit von den Messungen des Gassensors 12 in der Messkammer 11 wird das Spülen beendet und die zweite Pumpe 10 ausgeschaltet. Die zum Schluss in der Messkammer 11 ermittelten Parameter dienen als Startpunkt oder Nullpunkt für die weiteren Messungen.

Schritt 102: In der Anreicherungsphase wird die erste Pumpe 9 eingeschaltet, wodurch das Trägergas 16 in der Vorrichtung 1 befördert bzw. umgewälzt wird. Die zweite Pumpe 10 bleibt ausgeschaltet und bildet nun ein geschlossenes Ventil. Das Trägergas 16 wird in einem Kreislauf zwischen der Messkammer 11 und der Membran 13 bewegt. Da der Druck im Inneren des Hochspannungsgerätes 3 größer ist als der Druck in der Vorrichtung 1, gelangen Gase 4 aus dem Isoliermedium 2 durch die in eine Richtung gasmoleküldurchlässige Membran 13 in die Vorrichtung 1. Damit kommt es zu einer Anreicherung des Trägergases 16. Die Dauer der Anreicherung kann entweder durch eine fest vorgegebene Zeit oder über die Messungen des Gassensors 12 in der Messkammer 11 bestimmt werden.

Schritt 103: Nach der Anreicherungsphase werden die Menge und die Art der Gase 4 in der Messkammer 11 über den Gassensor 12 bestimmt. Nach der Bestimmung der Gase 4 wird die Vorrichtung 1 gespült.

Das beschriebene Verfahren kann entweder permanent oder aber einige Male pro Tag durchgeführt werden. Ein diskontinuierlicher Betrieb der Vorrichtung 1 kann zu einer Erhöhung der Lebenszeit des in der Messkammer 11 verwendeten Gassensors 12 führen.

### BEZUGSZEICHEN

- 1: Vorrichtung
- 2: Isoliermedium
- 3: Hochspannungsgerät
- 4: Gas
- 5: Einlass
- 6: Auslass
- 7: erste Leitung
- 8: zweite Leitung
- 9: erste Pumpe
- 10: zweite Pumpe
- 11: Messkammer
- 12: Gassensor
- 13: Membran
- 14: Thermoelement
- 15: Filter
- 16: Trägergas
- 17: Steuereinrichtung
- 18: Temperatursensor
- 19: erste Verbindungsleitung
- 20: zweite Verbindungsleitung

## Patentansprüche

1. Vorrichtung (1) zur Detektion von Gas (4) in einem mit einem Isoliermedium (2) gefüllten Hochspannungsgerät (3), aufweisend
- einen Einlass (5) zum Einleiten und einen Auslass (6) zum Abführen eines Trägergases (16);
- wenigstens einen Gassensor (12) zur Detektion eines Gases (4);
- eine erste Pumpe (9) zum Befördern des Trägergases (16) in einem Kreislauf der Vorrichtung (1);
- eine Membran (13), die zumindest aus wenigstens einem semipermeablen Werkstoff besteht, zumindest teilweise von dem Isoliermedium (2) umgeben ist und zumindest teilweise von dem Trägergas (16) angeströmt wird;
**dadurch gekennzeichnet, dass**
- eine zweite Pumpe (10) zum Hineinbefördern des Trägergases (16) in die Vorrichtung (1) und zum Herausbefördern des Trägergases (16) aus der Vorrichtung (1) vorgesehen ist;
- während des Betriebes der ersten Pumpe (9) die zweite Pumpe (10) die Funktion eines verschlossenen Ventils hat und während des Betriebes der zweiten Pumpe (10) die erste Pumpe (9) die Funktion eines verschlossenen Ventils hat.
- neben der ersten Pumpe (9) und der zweiten Pumpe (10) kein Ventil vorhanden ist, durch das das Trägergas (16) in die Vorrichtung (1) hinein oder aus der Vorrichtung (1) heraus befördert werden kann.

2. Vorrichtung nach Anspruch 1, wobei
- der Einlass (5) eine erste Leitung (7) aufweist.

3. Vorrichtung nach einem der vorigen Ansprüche, wobei
- die Membran (13) zumindest teilweise rohrartig und/oder zumindest teilweise schlauchartig ausgebildet ist;
- die Membran (13) zumindest teilweise spiralförmig und/oder zumindest teilweise mäanderförmig und/oder zumindest teilweise wendelförmig ausgebildet ist.

4. Vorrichtung nach einem der vorigen Ansprüche, wobei
- der Auslass (6) eine zweite Leitung (8) aufweist.

5. Vorrichtung nach einem der vorigen Ansprüche, zusätzlich aufweisend
- eine Messkammer (11), in der der Gassensor (12) angeordnet ist;
wobei
- in der Messkammer (11) wenigstens ein Thermoelement (14) und/oder wenigstens ein Temperatursensor (18) angeordnet ist;
- der Temperatursensor (18) und das Thermoelement (14) mit einer Steuereinrichtung (17) verbunden sind;
- das Thermoelement (17) anhand der Messungen des Temperatursensors (18) gesteuert wird.

6. Vorrichtung nach den Ansprüchen 2 und 4 und 5, wobei
- die Messkammer (11) mit der ersten Leitung (7) und über die Membran (13) und die zweite Leitung (8) mit dem Auslass (6) verbunden ist.

7. Vorrichtung nach den Ansprüchen 2 und 5, wobei
- die Messkammer (11) zwischen der ersten Leitung (7) und der Membran (13) angeordnet ist.

8. Vorrichtung nach den Ansprüchen 2 und 4, wobei
- die zweite Pumpe (10) in der ersten Leitung (7) oder in der zweiten Leitung (9) sitzt.

9. Vorrichtung nach dem vorigen Anspruch, wobei
- falls die zweite Pumpe (10) in der ersten Leitung (7) sitzt, dann hat die erste Leitung (7) ein Aspektverhältnis, das größer als das der zweiten Leitung (8) ist und/oder das größer als 100 oder 200 oder 500 oder 1000 oder 2000 oder 5000 oder 10.000 ist, und/oder hat sie einen Strömungswiderstand, der größer als der der zweiten Leitung (8) ist und/oder der dem einer Leitung mit konstantem Innendurchmesser und einem Aspektverhältnis entspricht, das größer als 100 oder 200 oder 500 oder 1000 oder 2000 oder 5000 oder 10.000 ist;
- falls die zweite Pumpe (10) in der zweiten Leitung (8) sitzt, dann hat die erste Leitung (7) ein Aspektverhältnis, das größer als das der ersten Leitung (7) ist und/oder das größer als 100 oder 200 oder 500 oder 1000 oder 2000 oder 5000 oder 10.000 ist, und/oder hat sie einen Strömungswiderstand, der größer als der der ersten Leitung (7) ist und/oder der dem einer Leitung mit konstantem Innendurchmesser und einem Aspektverhältnis entspricht, das größer als 100 oder 200 oder 500 oder 1000 oder 2000 oder 5000 oder 10.000 ist.

10. Vorrichtung nach Anspruch 5, wobei
- die erste Pumpe (9) in einer ersten Verbindungsleitung (19), die einen Ausgang der Messkammer (11) mit einem Eingang der Membran (13) verbindet, oder in einer zweiten Verbindungsleitung (20) sitzt, die einen Ausgang der Membran (13) mit einem Eingang der Messkammer (11) verbindet.

11. Vorrichtung nach den Ansprüchen 2 und 5, wobei
- die erste Leitung (7) zwischen der ersten Pumpe (9) und der Membran (13) in eine erste Verbindungsleitung (19), die einen Ausgang der Messkammer (11) mit einem Eingang der Membran (13) verbindet, oder zwischen der ersten Pumpe (9) und der Messkammer (11) in eine zweite Verbindungsleitung (20) mündet, die einen Ausgang der Membran (13) mit einem Eingang der Messkammer (11) verbindet.

12. Verfahren zur Detektion von Gas (4) in einem mit einem Isoliermedium (2) gefüllten Hochspannungsgerät (3), vorzugsweise mittels einer Vorrichtung (1) nach einem der vorigen Ansprüche, wobei
- in einem ersten Schritt eine Messkammer (11) mit einem Trägergas (16) gespült wird, indem das Trägergas (16) mittels einer zweiten Pumpe (10) in die Messkammer (11) hinein und aus der Messkammer (11) heraus befördert wird, ohne dass ein Ventil dabei durchströmt wird;
- in einem zweiten Schritt nach dem ersten Schritt das Trägergas (16) mittels einer ersten Pumpe (9) aus der Messkammer (11) heraus, durch eine Membran (13) hindurch und in die Messkammer (11) hinein befördert wird und dabei Gas (4), das sich im die Membran (13) anströmenden Trägergas (16) angereichert hat, in der Messkammer (11) detektiert wird;
- die Membran (13) zumindest aus wenigstens einem semipermeablen Werkstoff besteht und zumindest teilweise von dem Isoliermedium (2) umgeben ist;
- während des Betriebs der ersten Pumpe (9) die zweite Pumpe (10) die Funktion eines verschlossenen Ventils hat und während des Betriebs der zweiten Pumpe (10) die erste Pumpe (9) die Funktion eines verschlossenen Ventils hat.

13. Verfahren nach dem vorigen Anspruch, wobei
- beim Spülen das Trägergas (16) durch eine erste Leitung (7) angesaugt, durch die Messkammer (11) und die Membran (13) befördert und durch eine zweite Leitung (8) ausgeblasen wird.

14. Verfahren nach einem der Ansprüche 12 oder 13, wobei
- das Trägergas (16) über einen Filter (15) angesaugt wird;
- die Menge und/oder Art des Gases (4) in der Messkammer (11) bestimmt werden, bevor und/oder nachdem das Trägergas (16) heraus befördert wurde;
- die Menge und/oder Art des Gases (4) in der Messkammer (11) bestimmt werden, bevor und/oder nachdem das Trägergas (16) befördert wurde.

## Claims

1. Device (1) for detecting gas (4) in high-voltage apparatus (3) filled with an insulating medium (2), comprising:
- an inlet (5) for the introduction and an outlet (6) for the discharge of a carrier gas (16);
- at least one gas sensor (12) for detecting a gas (4);
- a first pump (9) for conveying the carrier gas (16) in circulation of the device (1);
- a membrane (13) which consists at least of at least one semipermeable material and which is at least partly surrounded by the insulating medium (2) and is at least partly subjected to a flow of the carrier gas (16); and
**characterized in that**,
- a second pump (10) for conveying the carrier gas (16) into the device (1) and for conveying the carrier gas (16) out of the device (1) is provided;
- during operation of the first pump (9) the second pump (10) has the function of a closed valve and during operation of the second pump (10) the first pump (9) has the function of a closed valve;
- next to the first pump (9) and the second pump (10) there is no valve through which the carrier gas (16) can be conveyed into the device (1) or out of the device (1).

2. Device according to the preceding claim, wherein
- the inlet (5) has a first line (7).

3. Device according to one of the preceding claims, wherein
- the membrane (13) is formed to be at least partly tubular and/or at least partly hose-like;
- the membrane (13) is formed to be at least partly spiral and/or at least partly meandering and/or at least partly helical.

4. Device according to any one of the preceding claims, wherein
- the outlet (6) has a second line (8).

5. Device according to any one of the preceding claims, additionally comprising
- a measuring chamber (11) in which the gas sensor (12) is arranged;
wherein
- at least one thermoelement (14) and/or at least one temperature sensor (18) is or are arranged in the measuring chamber (11);
- the temperature sensor (18) and the thermoelement (14) are connected with a control device (17);
- the thermoelement (17) is controlled on the basis of the measurements of the temperature sensor (18).

6. Device according to preceding claims 2 and 4 and 5, wherein
- the measuring chamber (11) is connected with the first line (7) and by way of the membrane (13) and the second line (8) with the outlet (6).

7. Device according to preceding claims 2 and 5, wherein
- the measuring chamber (11) is arranged between the first line (7) and the membrane (13).

8. Device according to preceding claims 2 and 4, wherein
- the second pump (10) is seated in the first line (7) or in the second line (9).

9. Device according to the preceding claim, wherein
- if the second pump (10) is seated in the first line (7) then the first line (7) has an aspect ratio which is greater than the second line (8) and/or greater than 100, 200, 500, 1,000, 2,000, 5,000 or 10,000 and/or it has a flow resistance which is greater than that of the second line (8) and/or which corresponds with that of a line with a constant inner diameter and an aspect ratio greater than 100, 200, 500, 1,000, 2,000, 5,000 or 10,000;
- if the second pump (10) is seated in the second line (8) then the second line (8) has an aspect ratio which is greater than the first line (7) and/or greater than 100, 200, 500, 1,000, 2,000, 5,000 or 10,000 and/or it has a flow resistance which is greater than that of the first line (7) and/or which corresponds with that of a line with a constant inner diameter and an aspect ratio greater than 100, 200, 500, 1,000, 2,000, 5,000 or 10,000.

10. Device according to preceding claim 5, wherein
- the first pump (9) is seated in a first connecting line (19) connecting an outlet of the measuring chamber (11) with an inlet of the membrane (13) or in a second connecting line (20) connecting an outlet of the membrane (13) with an inlet of the measuring chamber (11).

11. Device according to the preceding claims 2 and 5, wherein
- the first line (7) opens between the first pump (9) and the membrane (13) into a first connecting line (19), which connects an outlet of the measuring chamber (11) with an inlet of the membrane (13), or between the first pump (9) and the measuring chamber (11) into a second connecting line (20), which connects an outlet of the membrane (13) with an inlet of the measuring chamber (11).

12. Method of detecting gas (4) in high-voltage apparatus (3) filled with an insulating medium (2), preferably by means of a device (1) according to any one of the preceding claims, wherein
- in a first step a measuring chamber (11) is flushed with a carrier gas (16) in that the carrier gas (16) is conveyed by means of a second pump (10) into the measuring chamber (11) and out of the measuring chamber (11) without in that case flowing through a valve;
- in a second step after the first step the carrier gas (16) is conveyed by means of a first pump (9) out of the measuring chamber (11), through a membrane (13) and into the measuring chamber (11) and in that case gas (4), which has accumulated in the carrier gas (16) flowing against the membrane (13), is detected in the measuring chamber (11); and
- the membrane (13) consists at least of at least one semipermeable material and is surrounded at least partly by the insulating medium (2):
- during operation of the first pump (9) the second pump (10) has the function of a closed valve and during operation of the second pump (10) the first pump (9) has the function of a closed valve.

13. Method according to the preceding claim, wherein
- during the flushing the carrier gas (16) is sucked through a first line (7), conveyed through the measuring chamber (11) and the membrane (13) and purged through a second line (8).

14. Method according to one of the preceding method claims 12 or 13, wherein
- the carrier gas (16) is sucked up by way of a filter (15);
- the amount and/or kind of the gas (4) in the measuring chamber is or are determined before and/or after the carrier gas (16) was conveyed out;
- the amount and/or kind of the gas (4) in the measuring chamber is or are determined before and/or after the carrier gas (16) was conveyed.

## Revendications

1. Dispositif (1) de détection de gaz (4) dans un appareil haute tension (3) rempli d'un agent isolant (2) comportant :
- une entrée (5) permettant l'introduction d'un gaz porteur (16) et une sortie (6) permettant l'évacuation de ce gaz porteur (16),
- au moins un capteur de gaz (12) permettant la détection d'un gaz (4),
- une première pompe (9) permettant de refouler le gaz porteur (16) dans un circuit du dispositif (1),
- une membrane (13) réalisée au moins en au moins un matériau semi-perméable, au moins partiellement entourée par l'agent isolant (2) et au moins partiellement parcourue par le gaz porteur (16),
**caractérisé en ce qu'**
- il est prévu une seconde pompe (10) permettant de refouler le gaz porteur (16) dans le dispositif (1) et d'évacuer ce gaz porteur (16) de ce dispositif (1),
- pendant le fonctionnement de la première pompe (9) la seconde pompe (10) remplit la fonction d'une soupape fermée et pendant le fonctionnement de la seconde pompe (10) la première pompe (9) remplit la fonction d'une soupape fermée,
- outre la première pompe (9) et la seconde pompe (10) il n'est pas prévu de soupape au travers de laquelle le gaz porteur (16) pourrait être refoulé dans le dispositif (1) ou évacué de ce dispositif (1).

2. Dispositif conforme à la revendication 1,
dans lequel l'entrée (5) comporte une première conduite (7).

3. Dispositif conforme à l'une des revendications précédentes,
dans lequel :
- la membrane (13) est réalisée au moins partiellement en forme de tube et/ou au moins partiellement en forme de tuyau flexible,
- la membrane (13) est réalisée au moins partiellement en forme de spirale et/ou au moins partiellement en forme de méandres et/ou au moins partiellement en forme d'hélice.

4. Dispositif conforme à l'une des revendications précédentes,
dans lequel la sortie (6) comporte une seconde conduite (8).

5. Dispositif conforme à l'une des revendications précédentes, comprenant en outre :
- une chambre de mesure (11) dans laquelle est monté le capteur de gaz (12),
- dans la chambre de mesure (11) est(sont) installés au moins un thermo-élément (14) et/ou au moins un capteur de température (18),
- le capteur de température (18) et le thermo-élément (14) sont reliés à un dispositif de commande (17),
- le thermo-élément (14) est commandé sur le fondement des mesures du capteur de température (18).

6. Dispositif conforme à l'une des revendications 2, 4 et 5, dans lequel : la chambre de mesure (11) est reliée à la première conduite (7) et à la sortie (6) par l'intermédiaire de la membrane (13) et de la seconde conduite (8).

7. Dispositif conforme aux revendications 2 et 5,
dans lequel la chambre de mesure (11) est située entre la première conduite (7) et la membrane (13).

8. Dispositif conforme aux revendications 2 et 4,
dans lequel la seconde pompe (10) est située dans la première conduite (7) ou dans la seconde conduite (9).

9. Dispositif conforme à la revendication précédente, dans lequel :
- si la seconde pompe (10) est située dans la première conduite (7) cette première conduite (7) a un rapport de forme qui est supérieur à celui de la seconde conduite (8), et/ou qui est supérieur à 100 ou 200 ou 500 ou 1000 ou 2000 ou 5000 ou 10000, et/ou a une résistance à l'écoulement qui est supérieure à celle de la seconde conduite (8) et/ou correspond à celle d'une conduite ayant un diamètre interne constant et un rapport de forme qui est supérieur à 100 ou 200 ou 500 ou 1000 ou 2000 ou 5000 ou 10000,
- si la seconde pompe (10) est située dans la seconde conduite (8), cette seconde conduite (8) a un rapport de forme qui est supérieur à celui de la première conduite (7) et/ou qui est supérieur à 100 ou 200 ou 500 ou 1000 ou 2000 ou 5000 ou 10000, et/ou a une résistance à l'écoulement qui est supérieure à celle de la première conduite (7) et/ou correspond à celle d'une conduite ayant un diamètre interne constant et un rapport de forme qui est supérieur à 100 ou 200 ou 500 ou 1000 ou 2000 ou 5000 ou 10000.

10. Dispositif conforme à la revendication 5, dans lequel :
la première pompe (9) est située dans une première conduite de liaison (19) qui relie une sortie de la chambre de mesure (11) a une entrée de la membrane (13) ou est située dans une seconde conduite de liaison (20) qui relie une sortie de la membrane (13) avec une entrée de la chambre de mesure (11).

11. Dispositif conforme aux revendications 2 et 5, dans lequel :
la première conduite (7) débouche entre la première pompe (9) et la membrane (13) dans une première conduite de liaison (19) qui relie une sortie de la chambre de mesure (11) avec une entrée de la membrane (13) ou entre la première pompe (9) et la chambre de mesure (11) dans une seconde conduite de liaison (20) qui relie une sortie de membrane (13) avec une entrée de la chambre de mesure (11).

12. Procédé de détection de gaz (4) dans un appareil haute tension (3) rempli d'un agent isolant (2), de préférence au moyen d'un dispositif (1) conforme à l'une des revendications précédentes, selon lequel :
- dans une première étape, une chambre de mesure (11) est rincée avec un gaz porteur (16) du fait que ce gaz porteur (16) est refoulé dans la chambre de mesure (11) et extrait de cette chambre de mesure (11) au moyen d'une seconde pompe (10) sans circuler au travers d'une soupape,
- dans une seconde étape postérieure à la première étape, le gaz porteur (16) est au moyen d'une première pompe (9) extrait de la chambre de mesure (11) transféré au travers d'une membrane (13) et refoulé dans la chambre de mesure (11), et ainsi du gaz (4) qui s'est enrichi en gaz porteur (16) en traversant la membrane (13) est détecté dans la chambre de mesure (11),
- la membrane (13) est constituée d'au moins un matériau semi-perméable et est entourée au moins en partie par l'agent isolant (2),
- pendant le fonctionnement de la première pompe (9), la seconde pompe (10) remplit la fonction d'une soupape fermée et pendant le fonctionnement de la seconde pompe (10), la première pompe (9) remplit la fonction d'une soupape fermée.

13. Procédé conforme à la revendication précédente, selon lequel :
lors du rinçage, le gaz porteur (16) est aspiré dans une première conduite (7), refoulé au travers de la chambre de mesure (11) et de la membrane (13) et extrait par soufflage dans une seconde conduite (8).

14. Procédé conforme à l'une des revendications 12 et 13, selon lequel :
- le gaz porteur (16) est aspiré par l'intermédiaire d'un filtre (15),
- la quantité et/ou le type du gaz (4) est(sont) déterminé(es) dans la chambre de mesure (11) avant et/ou après l'extraction du gaz porteur (16) de celle-ci,
- la quantité et/ou le type de gaz (4) est(sont) déterminé(s) dans la chambre de mesure (11) avant et/ou après le refoulement du gaz porteur (16) de celle-ci.
